# EUROPÄISCHE PATENTANMELDUNG

(11) **EP 4 179 969 A1**
(43) Veröffentlichungstag der Anmeldung: **17.05.2023**
(21) Anmeldenummer: 22206336.4
(22) Anmeldetag: 09.11.2022
(51) Int. Cl.: A61B 5/0536, A61B 5/256, A61B 5/265, A61B 5/27, A61B 5/00

(54) **ELEKTRODENGURT UND VERWENDUNG EINES ELEKTRODENGURTS ZUR ELEKTROIMPEDANZTOMOGRAPHIE**

(30) Priorität: 16.11.2021 DE 102021212863
(71) Anmelder: ITP GmbH - Gesellschaft Für Intelligente Textile Produkte, 99423 Weimar (DE)
(72) Erfinder: HUSCHKE, Dirk, 99448 Rittersdorf (DE); RICHTER, Klaus, 99425 Weimar (DE)
(74) Vertreter: Liedtke & Partner Patentanwälte

(57) **Zusammenfassung**

Die Erfindung betrifft einen Elektrodengurt (1) aus einem elastisch dehnbaren textilen Material, umfassend eine obere Elektrodenreihe (2) von entlang einer Längsrichtung (L) äquidistant angeordneten Elektroden (4), eine in einer Querrichtung (Q) darunter angeordnete untere Elektrodenreihe (3) von entlang einer Längsrichtung (L) äquidistant angeordneten Elektroden (4), eine Kontaktleiterplatte (7) sowie einen Gurtverschluss (8). Die Elektroden (4) sind auf je einer Elektrodenleiterplatte (5) derart angeordnet, dass sie auf einer Elektrodenseite aus der Oberfläche des textilen Materials herausragen. Die Elektroden (4) sind mittels jeweils mindestens einer elastisch dehnbaren, flexiblen und in dem textilen Material eingearbeiteten Leiterbahn (6) elektrisch mit der Kontaktleiterplatte (7) verbunden. Die Kontaktleiterplatte (7) ist mit einem Übertragungsmittel zur Übertragung mindestens eines elektrischen Signals verbindbar.

Ferner betrifft die Erfindung eine Verwendung eines Elektrodengurts (1) zur Elektroimpedanztomographie (EIT) eines Körperteils.

## Beschreibung

Die Erfindung betrifft einen Elektrodengurt und dessen Verwendung zur Elektroimpedanztomographie.

Es ist bekannt, dass biologisches Gewebe einen charakteristischen, gewebetypabhängig unterschiedlichen spezifischen Wechselstromwiderstand (im Folgenden auch als Körperimpedanz oder Bioimpedanz bezeichnet) aufweist. Der Wechselstromwiderstand, der zwischen zwei auf einem Körper oder Körperteil angeordneten Elektroden gemessen wird, wird dabei durch sämtliche Gewebearten und Organe bestimmt, entlang denen ein Stromfluss zwischen den beiden Elektroden möglich ist.

Dieser Wechselstromwiderstand kann daher im Prinzip der Dämpfung eines Bündels von Röntgenstrahlen gleichgesetzt werden, das an einem Punkt in einen Körper eintritt und eine Vielzahl unterschiedlicher Gewebearten mit spezifischen Schwächungskoeffizienten durchläuft. In gleicher Weise wie bei einer radiologischen Bildgebung kann daher auch bei einer Körperimpedanzmessung mittels tomographischer Rekonstruktionsverfahren eine zweidimensionale Verteilung gewebespezifisch unterschiedlicher Impedanzwerte in einer Schnittebene durch einen Körper oder ein Körperteil ermittelt werden, wenn eine Impedanzmessung nicht nur zwischen zwei Elektroden, sondern zwischen einer Vielzahl von Elektroden durchgeführt wird, die bevorzugt kreisförmig in dieser Schnittebene angeordnet sind. Derartige, als Elektroimpedanztomographie (Electrical Impedance Tomography, EIT) bezeichnete Mess- und Rekonstruktionsverfahren sind bekannt und beispielsweise in der Veröffentlichung BH Brown (2003) Electrical impedance tomography (EIT): a review, Journal of Medical Engineering & Technology, 27:3, 97-108, DOI: 10.1080/0309190021000059687 beschrieben.

Das Kleben einer Vielzahl von Einzelelektroden ist arbeitsaufwändig, belastet den Patienten und erfordert hohe Sorgfalt, da die Rekonstruktionsalgorithmen empfindlich gegen Abweichungen der Elektrodenpositionen von der fiktiven gemeinsamen Schnittebene sind. Diese Schwierigkeiten vergrößern sich, wenn die Impedanzverteilung in einer Volumenrekonstruktion ermittelt werden soll, das heißt: nicht nur in einer einzigen Schnittebene, sondern in einer Mehrzahl von parallelen, möglichst äquidistant beabstandeten Schnittebenen. Daher besteht ein Bedarf an einer Vorrichtung zur verbesserten Anordnung von Elektroden für eine Körperimpedanzmessung und an einem Messverfahren zur impedanztomographischen Messung mittels einer solchen Elektrodenanordnung.

Aus der Veröffentlichung US 2019/0142299 A1 sind eine Vorrichtung und ein Verfahren zur Elektroimpedanztomographie mit einer mehrdimensional ausgedehnten Elektrodenanordnung bekannt. Damit rekonstruierte Bilder können Ebenen der mehrdimensional ausgedehnten Elektrodenanordnung sowie auch einem Bereich zugeordnet werden, der außerhalb der von den Elektroden aufgespannten Ebenen liegt. Eine Rekonstruktion von Bildern kann dadurch erfolgen, dass ein Finite-Elemente-Gitter mit mehreren Ebenen für die verschiedenen Bereiche zur Erzeugung von Bildern definiert wird.

Aus der Offenlegungsschrift DE 102 38 310 A1 ist eine insbesondere für die Elektroimpedanztomographie vorgesehene Elektrodenanordnung bekannt, die mehrere Elektroden zur elektrischen Kontaktierung eines Messobjekts und einen gürtelförmigen Elektrodenträger zur Umfassung des Messobjekts umfasst. Die Elektroden sind an dem gürtelförmigen Elektrodenträger angebracht und in dessen Längsrichtung positionierbar.

Das Dokument US 2020/0138335 A1 beschreibt eine Vorrichtung zur Elektroimpedanztomographie mit einem Feld von Elektroden, eine Messwerterfassungseinrichtung, einer Rechen- und Steuereinheit und einer Datenerfassungseinheit. Die Rechen- und Steuereinheit steuert die Erfassung und Verarbeitung von Elektroimpedanztomographie - Daten und ist zur Identifizierung eines Herzbereiches an einem Patienten eingerichtet.

Der Erfindung liegt die Aufgabe zu Grunde, einen Elektrodengurt zur verbesserten Anordnung von Elektroden für eine Körperimpedanzmessung anzugeben. Diese Aufgabe wird erfindungsgemäß durch einen Elektrodengurt mit den Merkmalen des Anspruchs 1 gelöst.

Ferner liegt der Erfindung die Aufgabe zu Grunde, eine Verwendung eines solchen Elektrodengurts für eine Elektroimpedanztomographie eines Körperteils anzugeben. Diese Aufgabe wird erfindungsgemäß durch eine Verwendung mit den Merkmalen des Anspruchs 8 gelöst.

Vorteilhafte Ausgestaltungen der Erfindung sind Gegenstand der Unteransprüche.

Ein Elektrodengurt aus einem elastisch dehnbaren textilen Material umfasst erfindungsgemäß äquidistant entlang einer Längsrichtung in einer oberen Elektrodenreihe angeordnete Elektroden und äquidistant entlang der Längsrichtung in einer unteren Elektrodenreihe angeordnete Elektroden. Die untere Elektrodenreihe ist entlang einer Querrichtung unterhalb der oberen Elektrodenreihe angeordnet. Längsrichtung und Querrichtung sind senkrecht zueinander.

Die Elektroden sind auf jeweils einer Elektrodenleiterplatte derart angeordnet, dass sie auf einer Elektrodenseite aus der Oberfläche des textilen Materials herausragen und eine Körperoberfläche kontaktieren, wenn der Elektrodengurt mit der Elektrodenseite auf die Körperoberfläche weisend um ein Körperteil fixiert wird.

Der Elektrodengurt umfasst ferner eine Kontaktleiterplatte, die mit einem Übertragungsmittel verbindbar ist, das zur Übertragung mindestens eines elektrischen Signals eingerichtet ist. Außerdem umfasst der Elektrodengurt einen Gurtverschluss, der dafür eingerichtet ist, den Elektrodengurt um ein Körperteil herum anzulegen und zu fixieren, wobei beim Fixieren der Elektrodengurt unter leichter Dehnung derart gespannt wird, dass die Elektroden auf die Oberfläche des umschlossenen Körperteils angedrückt werden.

Die Elektroden sind jeweils mittels mindestens einer elastisch dehnbaren flexiblen Leiterbahn elektrisch mit der Kontaktleiterplatte verbunden, wobei die flexible Leiterbahn in dem textilen Material eingearbeitet, beispielsweise eingewirkt, eingewebt oder eingeklebt ist.

Mit dem erfindungsgemäßen Elektrodengurt ist eine definierte, insbesondere näherungsweise äquidistante und in einer Schnittebene liegende Anordnung von Elektroden einer Elektrodenreihe möglich. Zudem sind Schnittebenen, die durch die obere, die untere und optional noch weitere, in gleichen Querabständen angeordnete Elektrodenreihen bestimmt werden, in guter Näherung parallel. Dadurch ist eine besonders exakte, sehr gut reproduzierbare impedanztomographische Messung und Rekonstruktion möglich.

Zudem ermöglicht der erfindungsgemäße Elektrodengurt eine besonders einfache und den Patienten wenig belastende Montage und Demontage aller zur Messung erforderlichen Elektroden und erlaubt dadurch eine Verkürzung der Untersuchungszeit und eine Durchführung einer genauen Messung auch durch weniger erfahrenes medizinisches Personal. Insbesondere entfällt durch die elastische Dehnbarkeit des Elektrodengurts die Notwendigkeit, eine Elektrodenanordnung für die individuellen anatomischen Verhältnisse bei einem Patienten anzupassen und/oder zu konfektionieren. Ein weiterer Vorteil des vorgeschlagenen Elektrodengurts besteht darin, dass durch die elastische Rückstellkraft des elastisch dehnbaren Materials die Elektroden zuverlässig am Körper des Patienten fixiert werden, wobei die Abstandsverhältnisse zwischen den Elektroden beibehalten werden. Dadurch können Rekonstruktionsverfahren zur Erzeugung von Schnittbildern wenigstens teilweise unabhängig von den individuellen anatomischen Verhältnissen angewendet werden.

Indem die Elektroden mittels elastisch dehnbaren, im textilen Material eingearbeiteten Leiterbahnen mit der Kontaktleiterplatte elektrisch verbunden sind, ist eine separate Leitungsführung von Elektrodenkabeln außerhalb des Elektrodengurts überflüssig. Dadurch wird sowohl die Handhabung des Elektrodengurts vereinfacht als auch die Unempfindlichkeit gegenüber beispielsweise induktiv oder kapazitiv einstreuenden Störungen verringert.

Bei einer Ausführungsform sind die entlang einer Leiterbahnrichtung elastisch dehnbaren flexiblen Leiterbahnen als Kupfer - Folienbahnen ausgebildet, bei denen mindestens eine Kupferbahn entlang der Leiterbahnrichtung mäanderförmig aufgefaltet in ein elastisch dehnbares, flexibles Folienmaterial eingelegt ist. Wenn der Elektrodengurt in der Längsrichtung auf Zug beansprucht wird, dehnen sich das elastisch dehnbare textile Material und damit auch das elastisch dehnbare flexible Folienmaterial, wobei die aufgefaltete Kupferbahn entlang der Leiterbahnrichtung gestreckt (das heißt: entfaltet) wird. Dadurch werden die einzelnen Elektroden von der Zugkraft entlastet und die relativen Abstände der Elektroden (bezogen auf die Länge des Elektrodengurts) werden beibehalten. Somit kann, unabhängig vom Umfang des umschlossenen Körperteils und von der bei der Fixierung des Elektrodengurts aufgebrachten Dehnung, eine besonders gut reproduzierbare Elektrodenanordnung gewährleistet werden.

Bei einer Ausführungsform sind die elastisch dehnbaren flexiblen Leiterbahnen in Leiterbahnrichtung um mindestens 25 Prozent, bevorzugt um mindestens 50 Prozent der ungedehnten Länge dehnbar. Ein so ausgebildeter Elektrodengurt ist besonders vielseitig einsetzbar, weil er zum Umschlingen von Körpern oder Körperteilen mit sehr unterschiedlichen Umfängen geeignet ist.

Bei einer Ausführungsform ist eine erste Elektrode mit der Kontaktleiterplatte entlang mindestens einer Elektrodenleiterplatte einer weiteren Elektrode verbunden, welche in Längsrichtung zwischen der ersten Elektrode und der Kontaktleiterplatte angeordnet ist. Bevorzugt sind in entlang einer Elektrodenreihe nur benachbarte Elektrodenleiterplatten mittels dehnbarer Leiterbahnen verbunden, wobei jede Elektrodenleiterplatte diejenigen Leiterbahnen durchschleift, welche Elektroden kontaktieren, die auf dem der Kontaktleiterplatte abgewandten Teil ihrer Elektrodenreihe angeordnet sind.

Bei dieser Ausführungsform können die Längen der dehnbaren Leiterbahnen kürzer und bevorzugt gleich lang gewählt werden, was die Dehnbarkeit und die Flexibilität des Elektrodengurts verbessert.

Bei einer Ausführungsform umfasst jede Elektrodenreihe mindestens zehn, bevorzugt mindestens 15 Elektroden, die in Längsrichtung äquidistant, bevorzugt in einem Abstand von mindestens 70 Millimeter und höchstens 90 Millimeter, angeordnet sind, wobei der Abstand benachbarter Elektrodenreihen in Querrichtung näherungsweise gleich dem Abstand der Elektroden einer Elektrodenreihe in Längsrichtung ist und wobei benachbarte Elektrodenreihen in Längsrichtung gegeneinander um den halben Abstand der Elektroden einer Elektrodenreihe in Längsrichtung versetzt sind.

Mit dieser Ausführungsform wird eine besonders gleichmäßige Elektrodenanordnung erzielt, die besonders gut für eine mindestens zwei Schnittebenen umfassende Elektroimpedanztomographie am menschlichen Oberkörper geeignet ist.

Bei einer Ausführungsform sind die Elektroden als Silber / Silberchlorid - Elektroden ausgebildet. Diese Ausführungsform ist in der Herstellung kostengünstig, weist unter Verwendung eines Kontaktgels einen geringen Elektrodenübergangswiderstand und eine hohe Störfestigkeit insbesondere gegenüber bewegungsinduzierten Artefakten auf.

Bei einer Ausführungsform ist der Gurtverschluss über mindestens eine auf der Elektrodenseite auf dem textilen Material angeordnete Klebefläche gebildet. Beim Verschließen haftet die mindestens eine Klebefläche auf der der Elektrodenseite gegenüberliegenden Oberflächenseite des Elektrodengurts. Mit dieser Ausführungsform kann ein Elektrodengurt besonders leicht fixiert und gelöst werden.

An Stelle der mindestens einen Klebefläche können auch korrespondierende Klettverschlussflächen auf die Elektrodenseite und auf die gegenüberliegende Oberflächenseite aufgebracht sein.

Bei der Verwendung eines Elektrodengurts nach einer der oben beschriebenen Ausführungsformen zur Elektroimpedanztomographie eines Körperteils eines menschlichen oder tierischen Körpers wird erfindungsgemäß der Elektrodengurt um den Körperteil derart umschlingend angeordnet, dass Elektroden einer Elektrodenreihe in jeweils einer Schnittebene durch den umschlungenen Körperteil liegen. Für jede Elektrodenreihe wird durch Impedanzmessung zwischen mindestens zwei gegenüberliegenden Elektroden und tomographische Rekonstruktion eine zweidimensionale Körperimpedanzwertverteilung in der zugeordneten Schnittebene ermittelt.

Aus der Mehrzahl der zweidimensionalen Körperimpedanzwertverteilungen in den Schnittebenen, welche den zur Messung herangezogenen Elektrodenreihen zugeordnet sind, wird eine dreidimensionale Körperimpedanzwertverteilung in dem von dem Elektrodengurt umschlossenen Volumenabschnitt des Körperteils bestimmt.

Die Vorteile dieser Verwendung entsprechen den bereits beschriebenen Vorteilen des erfindungsgemäßen Elektrodengurts. Insbesondere werden eine genauere, besser reproduzierbare Bestimmung der dreidimensionalen Körperimpedanzwertverteilung sowie eine einfachere und schnellere Messung ermöglicht.

Bei einer Ausführungsform der Verwendung wird der Elektrodengurt derart um einen Thorax geschlungen, dass jede Elektrodenreihe je eine Transversalebene durch den Thorax umschließt. Diese Ausführungsform eignet sich besonders gut zur thorakalen Elektroimpedanztomographie.

Ausführungsbeispiele der Erfindung werden im Folgenden anhand einer Zeichnung näher erläutert.

Darin zeigt:

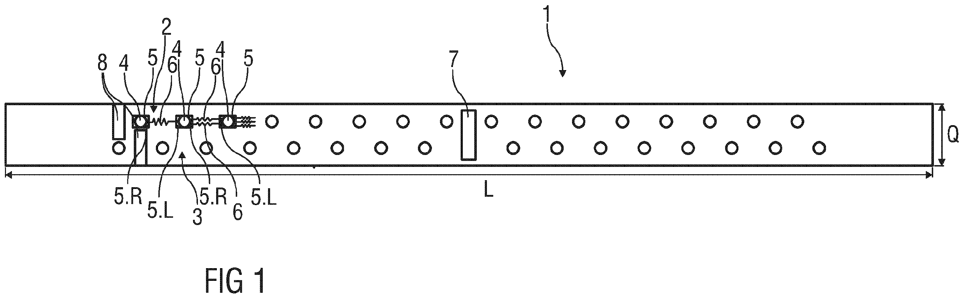

- Figur 1: schematisch einen Elektrodengurt zur Körperimpedanzmessung.

**Figur 1** zeigt schematisch einen Elektrodengurt 1 aus einem flexiblen, vorzugsweise elastisch dehnbaren Material, der in seiner Ausdehnung entlang einer Längsrichtung L für das Umfassen eines menschlichen Thorax bemessen ist. Beispielhaft hat der Elektrodengurt 1 eine Länge von etwa 1060 Millimeter.

Entlang einer Querrichtung Q weist der Elektrodengurt 1 beispielhaft eine Breite von etwa 70 Millimeter auf. Auf dem Elektrodengurt 1 sind eine obere und eine untere Elektrodenreihe 2, 3 von in Längsrichtung L gleichmäßig (äquidistant) mit einem Abstand von etwa 50 Millimeter beabstandeten Elektroden 4 angeordnet. Beispielhaft umfasst die obere Elektrodenreihe 2 16 Elektroden 4 und die untere Elektrodenreihe 3 ebenfalls 16 Elektroden 4. Die untere Elektrodenreihe 3 ist in Querrichtung Q unterhalb und in Längsrichtung L derart versetzt gegenüber der oberen Elektrodenreihe 2 angeordnet, dass eine Elektrode 4 der oberen Elektrodenreihe 2 jeweils in Längsrichtung L näherungsweise mittig zwischen zwei Elektroden 4 der unteren Elektrodenreihe 3 liegt, wobei in der unteren Elektrodenreihe 3 die mittige Position zwischen der achten und neunten Elektrode 4 ausgespart bleibt, an der eine nachfolgend noch genauer beschriebene Kontaktleiterplatte 7 angeordnet ist.

Beispielhaft sind die Elektrodenreihen 2, 3 vom oberen beziehungsweise unteren Rand des Elektrodengurts 1 in einem Abstand von jeweils näherungsweise 20 Millimeter angeordnet und weisen demzufolge in Querrichtung Q einen Abstand von näherungsweise 50 Millimeter auf. Die Elektroden 4 können beispielhaft kreisrund mit einem Durchmesser von etwa 3 Millimeter ausgebildet sein. Mit anderen Worten: je drei benachbarte Elektroden 4 bilden ein gleichseitiges Dreieck. Durch diese Anordnung der Elektroden 4 wird eine besonders gleichmäßige (das heißt: äquidistante) Kontaktierung des von dem Elektrodengurt 1 umschlungenen Thoraxbereiches erreicht.

Es ist möglich, in Querrichtung Q weitere Elektrodenreihen 2, 3 hinzuzufügen, um eine dichtere Abtastung und/oder die Abtastung eines breiteren Thoraxbereiches zu ermöglichen. Bevorzugt sind auch in derartigen Ausführungsformen die Elektroden 4 möglichst gleichmäßig (in näherungsweise gleichen Abständen) über dem Elektrodengurt 1 angeordnet.

Obgleich in Figur 2 nur an einige Elektroden 4 beispielhaft dargestellt, ist jede Elektrode 4 auf einer zugeordneten Elektrodenleiterplatte 5 angeordnet, die mindestens eine mit dieser Elektrode 4 elektrisch verbundene Kontaktstelle aufweist. Bevorzugt ist die Elektrodenleiterplatte 5 verdeckt in den Elektrodengurt 1 eingearbeitet, so dass nur die aufgesetzte Elektrode 4 aus dem Elektrodengurt 1 herausragt.

Die Elektrodenleiterplatte 5 kann, muss jedoch nicht aus einem flexiblen Leiterplattenmaterial gefertigt sein. Um die Flexibilität des Elektrodengurts 1 nur möglichst geringfügig zu beeinträchtigen, ist eine Elektrodenleiterplatte 5 knapp bemessen und ragt nur entlang der Längsrichtung L in einem linken Kontaktbereich 5.L und in einem rechten Kontaktbereich 5.R über den Umfang der aufgesetzten Elektrode 4 hinaus.

In den Kontaktbereichen 5.L, 5.R sind nicht näher dargestellte Kontaktstellen angeordnet, die zur elektrischen und mechanischen Verbindung der Elektrodenleiterplatte 5 mit je einer flexiblen Leiterbahn 6, beispielsweise mittels Löten, vorgesehen sind. Zur besseren Übersichtlichkeit ist in Figur 1 nur eine einzige flexible Leiterbahn 6 dargestellt, jedoch umfasst der Elektrodengurt 1 mindestens eine solche flexible Leiterbahn 6 für jede der Elektrodenleiterplatten 5.

Eine flexible Leiterbahn 6 kann beispielsweise als Kupfer-Folienbahn ausgeführt sein, die mäanderförmig oder ziehharmonikaartig aufgefaltet sind und in ihrer Längsausdehnung um etwa 25 Prozent bis 50 Prozent dehnbar sind. Bevorzugt sind die flexiblen Leiterbahnen 6 zur Kontaktierung der Elektrodenleiterplatten 5 in den Elektrodengurt 1 eingearbeitet, beispielsweise eingewebt oder eingewirkt. Dadurch wird vermieden, dass die einzelnen Elektroden 4 über eine Vielzahl von separat geführten Elektrodenkabeln angeschlossen werden müssen. Dies verbessert die Beweglichkeit des Patienten, erleichtert das Anlegen des Elektrodengurts 1 am Patienten und reduziert die Empfindlichkeit gegenüber kapazitiv und/oder induktiv einstreuenden Störungen.

Die flexiblen Leiterbahnen 6 verbinden je eine Elektrode 4 mit einer Kontaktleiterplatte 7, die in Längsrichtung L mittig auf dem Elektrodengurt 1 angeordnet ist. Die Kontaktleiterplatte 7 kann mit einem nicht näher dargestellten aufgesetzten Steckverbinder versehen sein, über den ein mehradriges externes Elektrodenkabel elektrisch mit den Elektroden 4 und mechanisch mit dem Elektrodengurt 1 verbunden werden kann. Alternativ kann an der Kontaktleiterplatte 7 ein solches mehradriges Elektrodenkabel auch fest und dauerhaft angeordnet sein. Alternativ ist auch die Anordnung eines mehrkanaligen Signalübertragungsmoduls zur drahtlosen Übertragung von Messwerten auf der Kontaktleiterplatte 7 möglich, wobei jeder Elektrode 4 jeweils ein Kanal zugeordnet ist.

Nachfolgend werden Ausführungsformen der Leitungsführung für die flexiblen Leiterbahnen 6 erläutert. In einer ersten Ausführungsform wird je eine flexible Leiterbahn 6 zwischen einer Elektrodenleiterplatte 5 und der Kontaktleiterplatte 7 angeschlossen und über die Kontaktleiterplatte 7 mit einer Ader eines mehradrigen externen Elektrodenkabels, alternativ mit einem Kanal eines mehrkanaligen Signalübertragungsmoduls, verbunden. Bei dieser Ausführungsform wird eine der Anzahl von Elektroden 4 entsprechende Anzahl von flexiblen Leiterbahnen 6 parallel durch den Elektrodengurt 1 geführt. Im Allgemeinen weisen diese flexiblen Leiterbahnen 6 abhängig von der Entfernung der zugeordneten Elektroden 4 von der Kontaktleiterplatte 7 unterschiedliche Längen auf. Bei dieser Ausführungsform können Kontaktbereiche 5.L, 5.R besonders klein ausgestaltet werden oder teilweise entfallen. Dadurch kann eine besonders hohe Flexibilität des Elektrodengurts 1 erreicht werden.

In einer zweiten Ausführungsform werden flexible Leiterbahnen 6 jeweils nur zwischen Kontaktbereichen 5.L, 5.R von Elektrodenleiterplatten 5 angeordnet, die entlang einer Elektrodenreihe 2, 3 benachbart sind. Eine flexible Leiterbahn 6, die von einer ersten Elektrodenleiterplatte 5 zu einer benachbarten zweiten Elektrodenleiterplatte 5 geführt ist, wird auf der zweiten Elektrodenleiterplatte 5 von der Kontaktstelle auf einem ersten, der ersten Elektrodenleiterplatte 5 benachbarten (linken oder rechten) Kontaktbereich 5.L, 5.R zu einer korrespondierenden Kontaktstelle auf dem gegenüberliegenden zweiten (rechten oder linken) Kontaktbereich 5.R, 5.L durchkontaktiert.

Auf diese Weise werden sämtliche Elektroden 4 einer Elektrodenreihe 2, 3 mit flexiblen Leiterbahnen 6 an die Kontaktleiterplatte 7 angeschlossen, die jeweils nur zwischen benachbarten Elektrodenleiterplatten 5 geführt werden. Entsprechend der Anzahl von Elektroden 4, die in Längsrichtung L auf der der Kontaktleiterplatte 7 abgewandten Seite einer Elektrode 4 liegen, werden zusätzlich zu der flexiblen Leiterbahn 6, die mit dieser Elektrode 4 verbunden ist, weitere flexiblen Leiterbahnen 6 von dem der Kontaktleiterplatte 7 zugewandten Kontaktbereich 5.L, 5.R weggeführt.

Ein Vorteil dieser Ausführungsform besteht darin, dass die flexiblen Leiterbahnen 6 eine vergleichsweise kurze und im Wesentlichen gleiche Länge aufweisen. Dadurch kann eine übermäßige und/oder ungleichmäßige Dehnung der flexiblen Leiterbahnen 6 beim Dehnen des Elektrodengurts 1 vermieden werden.

Der Elektrodengurt 1 ist mit einem Gurtverschluss 8 versehen, der die Fixierung des Elektrodengurts 1 in leicht gespanntem Zustand um den Thorax eines Patienten derart ermöglicht, dass die Elektroden 4 zur Herstellung eines guten elektrischen Kontakts leicht auf der Hautoberfläche angedrückt werden. Im vorliegend dargestellten Ausführungsbeispiel ist der Gurtverschluss über zwei Klebeflächen 8 gebildet, die an einem Längsende des Elektrodengurts 1 angeordnet sind.

Die Klebeflächen 8 erstrecken sich in Querrichtung Q von einem oberen beziehungsweise unteren Rand des Elektrodengurts 1 über eine Breite von etwa 40 Millimeter, das heißt: über die halbe Elektrodengurtbreite hinaus gehend. In Längsrichtung L erstrecken sich die Klebeflächen 8 über eine Länge von etwa 3 Millimeter. Um die sichere Fixierung sämtlicher Elektroden 4 zu ermöglichen, sind die Klebeflächen 8 vorteilhaft bei oder (in Längsrichtung L) vor den äußeren Elektroden 4 einer Elektrodenreihe 2, 3 angeordnet.

Alternativ zu den Klebeflächen 8 kann ein Gurtverschluss auch als Schnalle oder Gurtschließe ausgebildet sein.

Anzahl und Ausgestaltung der Elektroden 4 hängen von der Messaufgabe ab. Für eine Verwendung bei der Elektroimpedanztomographie sind zwei oder mehr Elektrodenreihen 2, 3 mit je mindestens 10, bevorzugt mindestens 15 Elektroden 4 vorteilhaft. Zur Messung des Wechselstromwiderstandes (Impedanzwertes) werden Elektrodenpaare aus je zwei Elektroden 4 einer Elektrodenreihe 2, 3 gebildet, die sich näherungsweise gegenüberstehen, wenn der Elektrodengurt 1 um den Thorax des Patienten fixiert ist. Durch Vermessung aller derart gebildeten Elektrodenpaare und anschließende Verrechnung der jeweils gemessenen Impedanzwerte beispielsweise mittels einer Radon - Transformation kann ein zweidimensionales Schnittbild bestimmt werden, das die gewebeabhängigen Impedanzunterschiede in der von der jeweiligen Elektrodenreihe 2, 3 umschlossenen Transversalebene durch den Thorax beschreibt.

Indem mehrere, entsprechend den Abständen der Elektrodenreihen 2, 3 in Querrichtung Q beabstandete Transversalebenen zusammengefasst werden, kann aus der Mehrzahl der zugeordneten transversalen Schnittbilder eine dreidimensionale Verteilung der gewebeabhängigen Impedanzunterschiede in dem gesamten, von dem Elektrodengürtel 1 umschlossenen thorakalen Teilvolumen ermittelt werden.

Für eine Verwendung bei der Elektroimpedanztomographie sind die Elektroden 4 bevorzugt als Silber / Silberchlorid Elektroden ausgebildet, die zur Herstellung eines möglichst niederohmigen Elektrodenübergangswiderstands auf der dem Patientenkörper zugewandten Oberfläche mit einem Kontaktgel beschichtet werden.

Alternativ können die Elektroden 4 auch als polarisierbare Elektroden, beispielsweise aus Edelstahl oder Platin ausgebildet sein. Mit derartigen Elektroden 4 versehen, eignet sich der Elektrodengürtel 1 beispielsweise auch für Messaufgaben in der Elektrokardiographie oder in der Elektromyographie.

### BEZUGSZEICHENLISTE

- 1: Elektrodengurt
- 2: obere Elektrodenreihe
- 3: untere Elektrodenreihe
- 4: Elektrode
- 5: Elektrodenleiterplatte
- 5.L, 5.R: linker, rechter Kontaktbereich
- 6: flexible Leiterbahn
- 7: Kontaktleiterplatte
- 8: Klebefläche, Gurtverschluss

- L: Längsrichtung
- Q: Querrichtung

## Patentansprüche

1. Elektrodengurt (1) aus einem elastisch dehnbaren textilen Material, umfassend
- eine obere Elektrodenreihe (2) von entlang einer Längsrichtung (L) äquidistant angeordneten Elektroden (4),
- eine in einer Querrichtung (Q) darunter angeordnete untere Elektrodenreihe (3) von entlang einer Längsrichtung (L) äquidistant angeordneten Elektroden (4),
- eine Kontaktleiterplatte (7) sowie
- einen Gurtverschluss (8),
- wobei die Elektroden (4) auf je einer Elektrodenleiterplatte (5) derart angeordnet sind, dass sie auf einer Elektrodenseite aus der Oberfläche des textilen Materials herausragen,
- wobei die Elektroden (4) mittels jeweils mindestens einer elastisch dehnbaren, flexiblen und in dem textilen Material eingearbeiteten Leiterbahn (6) elektrisch mit der Kontaktleiterplatte (7) verbunden sind und
- wobei die Kontaktleiterplatte (7) mit einem Übertragungsmittel zur Übertragung mindestens eines elektrischen Signals verbindbar ist.

2. Elektrodengurt (1) nach Anspruch 1, **dadurch gekennzeichnet, dass** die elastisch dehnbaren flexiblen Leiterbahnen (6) als Kupfer - Folienbahnen ausgebildet sind, bei denen jeweils mindestens eine entlang einer Leiterbahnrichtung mäanderförmig aufgefaltete Kupferbahn in ein elastisch dehnbares, flexibles Folienmaterial eingelegt ist.

3. Elektrodengurt (1) nach einem der vorhergehenden Ansprüche, **dadurch gekennzeichnet, dass** die elastisch dehnbaren flexiblen Leiterbahnen (6) in Leiterbahnrichtung um mindestens 25 Prozent, bevorzugt um mindestens 50 Prozent ihrer ungedehnten Länge dehnbar sind.

4. Elektrodengurt (1) nach einem der vorhergehenden Ansprüche, **dadurch gekennzeichnet, dass** eine erste Elektrode (4) mit der Kontaktleiterplatte (7) entlang mindestens einer Elektrodenleiterplatte (5) einer weiteren Elektrode (4) verbunden ist, die zwischen der ersten Elektrode (4) und der Kontaktleiterplatte (7) angeordnet ist.

5. Elektrodengurt (1) nach einem der vorhergehenden Ansprüche, **dadurch gekennzeichnet, dass** jede Elektrodenreihe (2, 3) mindestens 10, bevorzugt mindestens 15 Elektroden (4) umfasst, die in Längsrichtung (L) äquidistant, bevorzugt in einem Abstand von mindestens 70 Millimeter und höchstens 90 Millimeter, angeordnet sind, wobei der Abstand benachbarter Elektrodenreihen (2, 3) in Querrichtung (Q) näherungsweise gleich dem Abstand der Elektroden (4) einer Elektrodenreihe (2, 3) in Längsrichtung (L) ist und wobei benachbarte Elektrodenreihen (2, 3) in Längsrichtung (L) gegeneinander um den halben Abstand der Elektroden (4) einer Elektrodenreihe (2, 3) in Längsrichtung (L) versetzt sind.

6. Elektrodengurt (1) nach einem der vorhergehenden Ansprüche, **dadurch gekennzeichnet, dass** die Elektroden (4) als Silber / Silberchlorid - Elektroden ausgebildet sind.

7. Elektrodengurt (1) nach einem der vorhergehenden Ansprüche, **dadurch gekennzeichnet, dass** der Gurtverschluss (8) über mindestens eine auf der Elektrodenseite auf dem textilen Material angeordnete Klebefläche (8) gebildet wird.

8. Verwendung eines Elektrodengurts (1) nach einem der vorhergehenden Ansprüche zur Elektroimpedanztomographie eines Körperteils eines menschlichen oder tierischen Körpers, wobei
- der Elektrodengurt (1) den Körperteil derart umschlingend angeordnet wird, dass Elektroden (4) einer Elektrodenreihe (2, 3) in jeweils einer Schnittebene durch den Körperteil liegen,
- für jede Elektrodenreihe (2, 3) durch Impedanzmessung zwischen mindestens zwei gegenüberliegenden Elektroden (4) und tomographische Rekonstruktion eine zweidimensionale Körperimpedanzwertverteilung in der zugeordneten Schnittebene ermittelt wird und
- aus der Mehrzahl der Körperimpedanzwertverteilungen in den den Elektrodenreihen (2, 3) zugeordneten Schnittebenen eine dreidimensionale Körperimpedanzwertverteilung in dem von dem Elektrodengurt (1) umschlossenen Volumenabschnitt des Körperteils bestimmt wird.

9. Verwendung nach Anspruch 8, **dadurch gekennzeichnet, dass** der Elektrodengurt (1) derart um einen Thorax geschlungen wird, dass jede Elektrodenreihe (2, 3) je eine Transversalebene durch den Thorax umschließt.
